# EUROPEAN PATENT SPECIFICATION

(11) **EP 2 478 899 B1**
(45) Date of publication and mention of the grant of the patent: **09.05.2018**
(21) Application number: 10815493.1
(22) Date of filing: 14.09.2010
(51) Int. Cl.: A23L 33/175, A61K 31/198, A61K 8/44, A61P 17/00, A61P 17/16, A61P 35/00, A61P 37/02, A61P 37/08, A61Q 17/04

(54) **COMPOSITION FOR ALLEVIATING ULTRAVIOLET IRRADIATION-INDUCED DAMAGE**
ZUSAMMENSETZUNG ZUR LINDERUNG DURCH ULTRAVIOLETTSTRAHLUNG HERBEIGEFÜHRTER SCHÄDEN
COMPOSITION DESTINEE A ATTENUER LES DOMMAGES CAUSES PAR LES RAYONS ULTRAVIOLETS

(30) Priority: 14.09.2009 JP 2009211255; 28.09.2009 JP 2009223327
(43) Date of publication of application: 25.07.2012
(73) Proprietor: SHISEIDO COMPANY, LTD., Tokyo 104-8010 (JP)
(72) Inventor: ASHIDA, Yutaka, Yokohama-shi Kanagawa 236-0004 (JP); TOJO, Yosuke, Yokohama-shi Kanagawa 236-0004 (JP); MIZUMOTO, Chieko, Yokohama-shi Kanagawa 236-0004 (JP); MITA, Masashi, Tokyo 104-8010 (JP)
(74) Representative: Henkel, Breuer & Partner
(86) International application number: PCT/JP2010/065789
(87) International publication number: WO 2011/030903

(56) References cited:
- EP-A1- 2 484 353
- WO-A1-99/63989
- WO-A1-2006/068133
- JP-A- 7 233 046
- JP-A- 11 049 630
- JP-A- 11 060 435
- JP-A- 57 158 724
- JP-A- 2007 070 343
- BURKE K E ET AL: "EFFECTS OF TOPICAL AND ORAL VITAMIN E ON PIGMENTATION AND SKIN CANCER INDUCED BY ULTRAVIOLET IRRADIATION IN SKH:2 HAIRLESS MICE", NUTRITION AND CANCER, TAYLOR & FRANCIS GROUP, GB, vol. 38, no. 1, 1 January 2000 (2000-01-01), pages 87-97, XP009051519, ISSN: 0163-5581, DOI: 10.1207/S15327914NC381_13
- BURKE KAREN E ET AL: "The effect of topical L-selenomethionine on minimal erythema dose of ultraviolet irradiation in humans", PHOTODERMATOLOGY PHOTOIMMUNOLOGY AND PHOTOMEDICINE, vol. 9, no. 2, 1992, pages 52-57, XP009177853, ISSN: 0905-4383
- SCHMUTZ J L: "Porphyria", ANNALES DE DERMATOLOGIE ET DE VENEREOLOGIE, vol. 134, no. 5, Part 2, May 2007 (2007-05), pages S73-S80, XP009177851, ISSN: 0151-9638
- BONAMONTE D ET AL: "Solar erythema: Clinical and pathogenetic considerations", ANNALI ITALIANI DI DERMATOLOGIA ALLERGOLOGICA CLINICA E SPERIMENTALE 200509 IT, vol. 59, no. 3, September 2005 (2005-09), pages 128-131, XP009177852, ISSN: 1592-6826
- POSTON ET AL: "Response of rainbow trout to source and level of supplemental dietary methionine", COMPARATIVE BIOCHEMISTRY AND PHYSIOLOGY. PART A. COMPARATIVE PHYSIOLOGY, ELSEVIER SCIENCE LTD, US, vol. 83, no. 4, 1 January 1986 (1986-01-01), pages 739-744, XP025218490, ISSN: 0300-9629, DOI: 10.1016/0300-9629(86)90720-6 [retrieved on 1986-01-01]
- THORNFELDT: "COSMECEUTICAL CONTAINING A NOVEL BOTANICAL BLEND REDUCES UV-INDUCED ERYTHEMA", COSMETIC DERMATOLOGY, KNOLLS PUB. GROUP, CEDAR KNOLLS, NJ, US, vol. 17, no. 10, 1 October 2004 (2004-10-01), pages 663-667, XP009071665, ISSN: 1041-3766
- BURKE, K. E.: 'L-selenomethionine and UV-induced skin damage' COSMETICS AND TOILETRIES vol. 107, no. 7, 1992, pages 51 - 61, XP008151788
- RAFFERTY, T. S. ET AL.: 'Differential expression of selenoproteins by human skin cells and protection by selenium from UVB-radiation- induced cell death' BIOCHEMICAL JOURNAL vol. 332, 1998, pages 231 - 236, XP008151796
- MASAYA TAKUMIDA: 'Series Kyoiku Koza 'Massho Zenteiki kara Zentei Shinkeikaku e -Saikin no Chiken-' 2. Massho Zenteiki Shogai ni Okeru Free Radical' EQUILIBRIUM RESEARCH vol. 64, no. 2, 2005, pages 43 - 49, XP008153489
- TAKUMIDA, M. ET AL.: 'Neuroprotection of vestibular sensory cells from gentamicin ototoxicity obtained using nitric oxide synthaseinhibitors, reactive oxygen species scavengers, brain-derived neurotrophic factors and calpain inhibitors.' ACTA OTOLARYNGOL vol. 123, 2003, pages 8 - 13, XP008038195

## Description

### TECHNICAL FIELD

The present invention relates to a composition for use in preventing or treating an ultraviolet irradiation-induced skin disease, the composition comprising one or more compounds selected from a group consisting of D-methionine and/or salts thereof. The invention further relates to the use of a composition as defined above for improving an ultraviolet irradiation-induced cosmetic skin condition, and as a sunscreen agent.

### Background Art

Ultraviolet rays are classified into long wavelength region ultraviolet rays of longer than about 320 nm (UV-A), medium wavelength region ultraviolet rays of about 320 to about 280 nm (UV-B) and short wavelength region ultraviolet rays of shorter than about 280 nm (UV-C) . Among these, the UV-C is not contained in solar lights reaching the ground since it is absorbed by ozone layer. While both of the UV-A and the UV-B are contained in the solar light reaching the ground, the UV-B is partly absorbed by the ozone layer. However, since the UV-A is not absorbed by the ozone layer, it is predominant in the ultraviolet rays reaching the ground, resulting in a skin damage.
Non-Patent Literature 1 discloses diseases in which the ultraviolet rays are implicated, including wrinkles, erythema, xeroderma pigmentosum, chronic actinic dermatitis, squamous cell carcinoma, basal cell carcinoma, malignant melanoma, Bowen disease, solar keratosis, photodermatosis, hydroa vacciniforme and photocontact dermatitis, while Non-Patent Literature 2 exemplifies solar dermatitis, chronic actinic dermatopathy, actinic keratosis, actinic cheilitis, Favre-Racouchot syndrome, photodermatosis, photocontact dermatitis, berloque dermatitis, photosensitive drug eruption, polymorphous light eruption, hydroa vacciniforme, solar urticaria, chronic photosensitive dermatitis, xeroderma pigmentosum, ephelides, porphyria, pellagra, Hartnup disease, solar keratosis, dermatomyositis, lichen planus, Darier disease, pityriasis rubra pilaris, rosacea, atopic dermatitis, chloasma, herpes simplex and lupus erythematosus.

### PRIOR ART DOCUMENTS

### Non-Patent Documents

Non-Patent document 1: "HIFUSHIKKAN SAISHIN NO CHIRYO (Latest methods for treating dermal diseases)", 2005-2006 (Nankodo Co. , Ltd.)
Non-Patent document 2: "HYOJUN HIFUKAGAKU (Standard dermatology)", 7th edition (Igaku-Shoin Ltd.)

### Disclosure of the Invention

### PROBLEM TO BE SOLVED BY THE INVENTION

Known conventional agents for preventing and/or treating an ultraviolet irradiation-induced skin damage include an ultraviolet scattering agent which inhibits absorption of the ultraviolet by a skin, such as titanium oxide, an ultraviolet absorber, such as ethyl hexyl p-methoxycinnamic acid, or an antioxidant which scavenge a free radical generated by the ultraviolet. The ultraviolet scattering agent or the ultraviolet absorber is, however, not used on a daily basis although it is effective outdoor in preventing sunburn. The antioxidant is problematic in terms of stability and safety. In addition, known agents for treating the ultraviolet irradiation-induced skin damages are limited only to symptomatic agents for treating. Accordingly, it is required to develop a composition for alleviating ultraviolet irradiation-induced damage, an external preparation for the skin, an anti-wrinkle agent, a sunscreen agent, a pharmaceutical composition for treating and/or preventing skin diseases, or a food composition, which can be used on a daily basis and is stable and safe.

### MEANS FOR SOLVING THE PROBLEM

The present invention is defined in the claims and, in one aspect, provides a composition for use in preventing or treating an ultraviolet irradiation-induced skin disease, the composition comprising one or more compounds selected from a group consisting of D-methionine and/or salts thereof.

In another aspect, the present invention relates to the use of a composition comprising one or more compounds selected from a group consisting of D-methionine and/or salts thereof for improving an ultraviolet irradiation-induced cosmetic skin condition.

In yet another aspect, the present invention relates to the use of a composition comprising one or more compounds selected from a group consisting of D-methionine and/or salts thereof as a sunscreen agent.

The composition for use in the invention may be in the form of an external preparation for the skin or in the form of a food product.

The skin disease described above may be selected from a group consisting of erythema, solar dermatitis, chronic actinic dermatopathy, actinic keratosis, actinic cheilitis, Favre-Racouchot disease, photodermatosis, photocontact dermatitis, berloque dermatitis, photosensitive drug eruption, polymorphous light eruption, hydroa vacciniforme, solar urticaria, chronic photosensitive dermatitis, xeroderma pigmentosum, ephelides, porphyria, pellagra, Hartnup disease, solar keratosis, dermatomyositis, lichen planus, Darier disease, pityriasis rubra pilaris, rosacea, atopic dermatitis, chloasma, herpes simplex, lupus erythematosus, squamous cell carcinoma, basal cell carcinoma and Bowen disease. The cosmetic skin condition described above may be wrinkles.

Disclosed herein is a method for treating and/or preventing an ultraviolet irradiation exposure-induced skin disease, comprising a step of administering a composition comprising one or more compounds selected from a group consisting of D-methionine and/or salts thereof. The skin disease described above may be selected from a group consisting of erythema, solar dermatitis, chronic actinic dermatopathy, actinic keratosis, actinic cheilitis, Favre-Racouchot disease, photodermatosis, photocontact dermatitis, berloque dermatitis, photosensitive drug eruption, polymorphous light eruption, hydroa vacciniforme, solar urticaria, chronic photosensitive dermatitis, xeroderma pigmentosum, ephelides, porphyria, pellagra, Hartnup disease, solar keratosis, dermatomyositis, lichen planus, Darier disease, pityriasis rubra pilaris, rosacea, atopic dermatitis, chloasma, herpes simplex, lupus erythematosus, squamous cell carcinoma, basal cell carcinoma and Bowen disease.

Further disclosed herein is a method for treating and/or preventing an ultraviolet irradiation exposure-induced skin disease, comprising a step of administering a composition comprising one or more compounds selected from a group consisting of D-methionine and/or salts thereof except for oral compositions. The skin disease described above may be selected from a group consisting of erythema, solar dermatitis, chronic actinic dermatopathy, actinic keratosis, actinic cheilitis, Favre-Racouchot disease, photodermatosis, photocontact dermatitis, berloque dermatitis, photosensitive drug eruption, polymorphous light eruption, hydroa vacciniforme, solar urticaria, chronic photosensitive dermatitis, xeroderma pigmentosum, ephelides, porphyria, pellagra, Hartnup disease, solar keratosis, dermatomyositis, lichen planus, Darier disease, pityriasis rubra pilaris, rosacea, atopic dermatitis, chloasma, herpes simplex, lupus erythematosus, squamous cell carcinoma, basal cell carcinoma and Bowen disease.

Further disclosed herein is a method for improving an ultraviolet irradiation-induced cosmetic skin condition, comprising a step of administering a composition comprising one or more compounds selected from a group consisting of D-methionine and/or salts thereof. In the method for improving a cosmetic skin condition described above, the composition may be an external preparation for the skin or a food composition.

Further disclosed herein is a method for improving an ultraviolet irradiation-induced cosmetic skin condition, comprising a step of administering a composition comprising one or more compounds selected from a group consisting of D-methionine and/or salts thereof except for oral compositions. In the method for improving a cosmetic skin condition described above, the composition may be an external preparation for the skin.

In the method for improving an ultraviolet irradiation-induced cosmetic skin condition, the improvement of a cosmetic skin condition includes, but is not limited to, an anti-wrinkle treatment and/or a sunscreen treatment.

As used herein, "salt" of D-methionine refers to any salt including metal salts and amine salts, provided that the ultraviolet irradiation-induced damage alleviating effect of D-methionine is not impaired. The metal salt described above may include an alkaline metal salt, an alkaline earth metal salt and the like. The amine salt described above may include triethylamine salt, benzylamine salt and the like.

As used herein, "derivative" of D-methionine refers to a D-methionine molecule bound covalently to any substituent group at its amino group, carboxyl group or side chain, provided that the alleviating effect of D-methionine on ultraviolet irradiation-induced damage is not impaired. The substituent group mentioned above includes, but is not limited to, a protective group, such as N-phenylacetyl group, 4,4'-dimethoxytrityl (DMT) group,etc.; a biological macromolecule , such as a protein, a peptide, a saccharide, a lipid, a nucleic acid, etc.; a synthetic polymer , such as a polystyrene, a polyethylene, a polyvinyl, a polyester,etc.; and a functional group such as an ester group, etc. The ester group mentioned above may include, for example, a methyl ester, an ethyl ester, other aliphatic ester or aromatic ester.

Since an amino acid exists as an optical isomer which is in either of an L-form or a D-form but a natural protein is made of L-amino acids bound via peptide bonds and only L-amino acids are employed excluding some exceptions such as a bacterial cell wall, it has been considered that in a mammal including a human only L-amino acids are present and utilized (Kinouchi, T. et al., TANPAKUSHITSU KAKUSAN KOSO (PROTEINS, NUCLEIC ACIDS AND ENZYMES), 50:453-460(2005), Lehninger Principles of Biochemistry [Vol.1] 2nd ed., pp132-147(1993), Japanese-language translation, Hirokawa Publishing Co., Harper's Biochemistry, Original version, 22nd ed., pp21-30(1991), Japanese-language translation, Maruzen Co., Ltd.). Accordingly, only L-amino acids have mostly been employed as amino acids academically and industrially for a long time.

Exceptional cases where a D-amino acid is employed include, for example, a case of using as a raw material for an antibiotics produced by a microorganism and a case of a food additive employing a D-amino acid in a DL-amino acid mixture just for the purpose of reducing cost of fractionating only an L-amino acid from a mixture of the L-amino acid and the D-amino acid. However, there has been no case of utilizing only an L-amino acid-free D-amino acid industrially as a bioactive substance.

D-serine and D-aspartic acid have been studied to a comparatively advanced stage because of their higher ratios of D-forms. D-serine is localized in a cerebrum and a hippocampus, and is known to be involved in an NMDA receptor modulator in the brain. D-aspartic acid is demonstrated to be localized in a testis and a pineal body, and reported to be involved in the regulation of hormone secretion (Japanese Patent Unexamined Publication No.2005-3558). However, the physiological effects of D-serine and D-aspartic acid on the skin has not been elucidated.

As indicated in Examples described below, D-methionine has not been known so far to have an alleviating effect on ultraviolet irradiation-induced damage. Therefore, the use of a composition comprising D-methionine as described herein is a novel invention.

Recently, it was reported that ddY mice had been allowed to access a 10 mM aqueous solution of a D-amino acid for 2 weeks and then determined for the D-amino acid concentration in each organ, which was 3 to 1000 pmol per gland in a pineal body and 2 to 500 nmol per wet gram in a brain tissue (Morikawa, A. et al., Amino Acids, 32: 13-20 (2007)). Based on this report, the lower limit of daily dose of D-methionine contained in a composition disclosed herein was calculated.

As indicated in Examples described below, D-methionine relevant to the present invention exhibits an alleviating effect on ultraviolet irradiation-induced damage at a concentration ranging from 0.001 to 100 µM (micro-molar) on a cultured human fibroblast. Accordingly, the amount of D-methionine contained in a pharmaceutical composition, anti-wrinkle agent, sunscreen agent and external preparation for the skin disclosed herein may be varied in a wide range, provided that D-methionine is delivered to a fibroblast in an in vivo skin tissue at a concentration range specified above. When the composition is an external preparation, the D-methionine content may be 0.0000015% by mass to 50% by mass in the entire amount of the composition or up to the maximum mass concentration capable of being formulated. Thus, when the composition described above is an external preparation, the D-methionine content is desirably 0.000003% by mass to 30% by mass, most desirably 0.00003% by mass to 3% by mass. The lower limit of the daily dose of D-methionine contained in the composition may be 0.01 ng, preferably 0.1 ng, more preferably 1 ng per kg body weight.

The pharmaceutical composition disclosed herein may further comprise one or more pharmaceutically acceptable additives, in addition to D-methionine and/or D-methionine salts, provided that the alleviating effect of D-methionine on ultraviolet irradiation-induced damage is not impaired. Such an additive includes, but is not limited to, a diluent, a swelling agent, a binder, an adhesive, a lubricant, a glidant, a plasticizer, a disintegrant, a carrier solvent, a buffering agent, a colorant, a flavoring agent, a sweetener, a preservative, a stabilizer, an adsorbent, as well as other pharmaceutical additives known to those skilled in the art.

An anti-wrinkle agent and/or sunscreen agent disclosed herein can be prepared using only D-methionine and/or salts of D-methionine. However, other components employed in external preparations for the skin such as cosmetic and pharmaceutical products including quasi drugs may appropriately be formulated as required to the extent that the effect of the invention is not impaired. Such other components (optionally formulated components) include, for example, oils, surfactants, powders, colorants, water, alcohols, thickening agents, chelating agents, silicones, antioxidants, UV absorbers, humectants, flavoring agents, various medicinal ingredients, preservatives, pH adjusters, neutralizer like that.

The external preparation for the skin disclosed herein may be any of those employed conventionally in an external preparation for the skin and a cosmetic composition, such as an ointment, a cream, an emulsion, a lotion, a pack, a bath salt and the like, and their dosage forms are not specified particularly.

To the external preparation for the skin disclosed herein, other components employed in external preparations for the skin such as cosmetic and pharmaceutical products including quasi drugs may appropriately be formulated as required, provided that the alleviating effect of D-methionine on ultraviolet irradiation-induced damage is not impaired. Such other components (optionally formulated components) include, for example, oils, surfactants, powders, colorants, water, alcohols, thickening agents, chelating agents, silicones, antioxidants, UV absorbers, humectants, flavoring agents, various medicinal ingredients, preservatives, pH adjusters, neutralizer.

The food composition disclosed herein may be any of those employed conventionally in a food composition, such as a beverage, a gummy candy, a candy, a tablet sweet, to which it is not limited.

### BRIEF DESCRIPTION OF THE DRAWINGS

Figure 1 is a graph showing the effect of L- or D-methionine addition before an ultraviolet irradiation in normal human dermal fibroblasts.
Figure 2 is a graph showing the effect of D-methionine addition before an ultraviolet irradiation in normal human dermal fibroblasts.
Figure 3 is a graph showing the effect of D-methionine addition after an ultraviolet irradiation in normal human dermal fibroblasts.
Figure 4 is a graph showing the effect of L- or D-serine addition before an ultraviolet irradiation in normal human dermal fibroblasts.
Figure 5 is a graph showing the effect of D-serine addition before an ultraviolet irradiation in normal human dermal fibroblasts.
Figure 6 is a graph showing the effect of D-serine addition after an ultraviolet irradiation in normal human dermal fibroblasts.
Figure 7 is a graph showing the effect of D-cycloserine addition after an ultraviolet irradiation in normal human dermal fibroblasts.

### DESCRIPTION OF EMBODIMENTS

Examples of the present invention described below are intended only to exemplify the invention rather than to limit the scope thereof.

All documents cited herein are incorporated by reference in its entirety.

### Example 1

### Alleviating effect of methionine on ultraviolet irradiation-induced damage

### Methods

### Cell culture

The cell employed was a commercially available human neonatal dermal fibroblast (Cryo NHDF-Neo, Sanko-Junyaku Co., Ltd.). This cell was inoculated at 2 × 10³ cells/mL to a commercially available culture dish of 35 mm in diameter (BD FALCON 353001, Becton Dickinson Japan), where it was cultured in a commercially available cell culture medium (D-MEM (1 g/L glucose, Wako Pure Chemical Industries, Ltd.) supplemented with 10% fetal bovine serum (hereinafter, referred to as "normal medium"). The cell described above may be cultured in the normal medium described above supplemented with an antibiotic (15240-062, Gibco) at 1%. This cell was cultured for about 24 hours in a 5% CO₂ and saturated water vapor atmosphere at 37°C (degrees Celsius) .

Thereafter, the culture medium for culturing the cell described above was switched to 1 mL of a BSO medium supplemented with a glutathione biosynthesis inhibitor BSO (L-buthionine-(S,R)-sulfoximine, Wako Pure Chemical Industries, Ltd.) at 1 × 10⁻³%, where the culture was conducted for about 24 hours in a 5% CO₂ and saturated water vapor atmosphere at 37°C (degrees Celsius). The BSO medium described above was prepared by a 200-fold dilution of a stock solution containing 0.2% BSO dissolved in ethyl alcohol with the normal medium described above.

### Addition of amino acid before ultraviolet irradiation

For determining the effect of adding methionine before ultraviolet irradiation (hereinafter, referred to as "pre-irradiation addition of methionine"), the culture medium was switched to a BSO medium added with 0.0001 to 100 µM (micro-molar) L-methionine (131-01603, Wako Pure Chemical Industries Ltd.) or D-methionine (2807, Peptide Institute Inc.) 24 hours before the irradiation. The ultraviolet irradiation after switching into a medium added with 0.1 µM (micro-molar) D-proline (165-14671, Wako Pure Chemical Industries Ltd.) was employed as a positive control, while the ultraviolet irradiation to the medium still being free of such an added amino acid was employed as a negative control.

### UV Irradiation medium

Ferric chloride (II) was dissolved in a distilled water at 2 × 10⁻³%, and the resultant solution was subjected to a 200-fold dilution (final concentration: 1 × 10⁻⁵%) with a phosphate buffered saline PBS (+) containing calcium ion and magnesium ion to obtain a medium (hereinafter, referred to as "UV irradiation medium"), which was warmed preliminarily to 37°C before use.

### UV Irradiation

Before UV-A irradiation, the culture medium was replaced with 1 mL of the UV irradiation medium described above. The UV-A irradiation was conducted using a UV light uniform exposure device UVE-502S+EL-160 (SAN-EI ELECTRIC) by irradiating a UV ray of 320 nm to 400 nm at 8 J/cm² and 9 J/cm² from about 20 cm above a culture dish in a state where the lid of the respective culture dish was removed. The UV dose was measured using UV RADIOMETER UVR-3036/S (Topcon Corporation).

### Addition of amino acid after ultraviolet irradiation

After UV-A irradiation at 8 J/cm², the medium was returned to the normal medium described above, and the culture was conducted in a 5% CO₂ and saturated water vapor atmosphere at 37°C (degrees Celsius) for 40 hours. For determining the effect of adding methionine after UV irradiation (hereinafter, referred to as "post-irradiation addition of methionine"), this 40-hour cultured medium was added with 0.001 to 100 µM (micro-molar) L- or D-methionine. The ultraviolet irradiation after switching into a medium added with 0.1 µM (micro-molar) D-proline was employed as a positive control, while the ultraviolet irradiation to the medium still being free of such an added amino acid was employed as a negative control.

### Cell damage measurement of pre- and post-irradiation additions

Thereafter, the culture medium was supplemented with Alamar Blue (trade mark, Biosource, Biosource International Inc. and Invitrogen) at a final concentration of 10%, and its supernatant was determined for the fluorescent intensity three hours later with an excitation wavelength of 544 nm and a fluorescent wavelength of 590 nm as described by Ahmed S.A. et al. J. Immunol. Method. 170, 211-224 (1994)) and in accordance with the manufacture's instruction. The percentage of the viable cell was obtained as a percentage of a quotient calculated by dividing the fluorescent intensity of Alamar Blue under each experimental condition by the fluorescent intensity in the negative control group containing no added amino acid.

### Results of pre-irradiation addition of methionine (1)

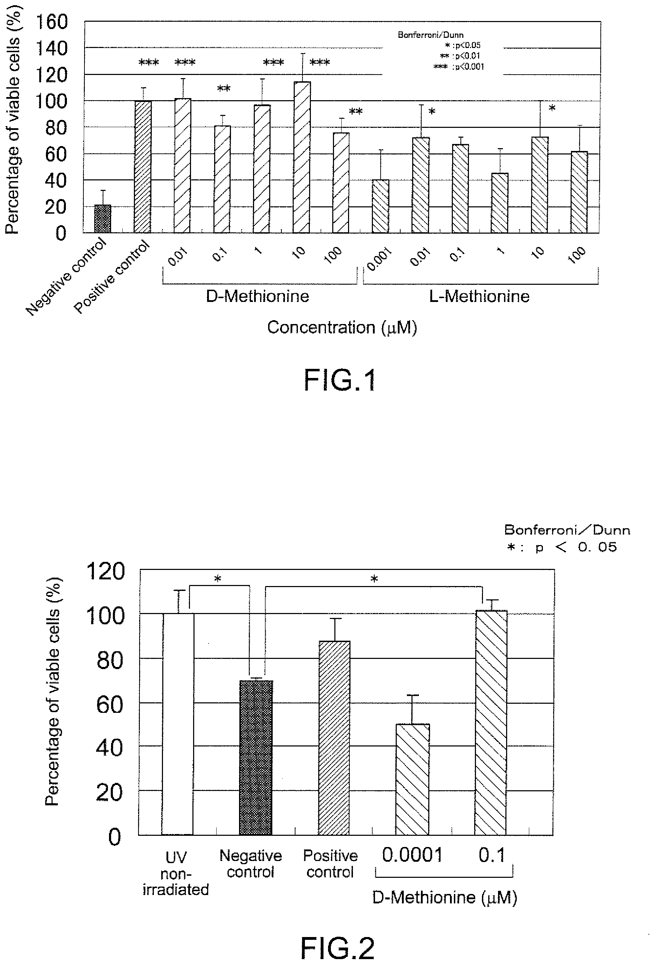

Figure 1 shows the results of the experiment examining the effect of L- or D-methionine on the damage of the fibroblast induced by the ultraviolet irradiation with the UV-A at 9 J/cm². The error bars for relevant experimental conditions are the standard deviations of the measured values of the results of the experiments repeated four times under the same conditions. The asterisk (*) indicates P<5%, asterisk (**) indicates P<1% and asterisk (***) indicates P<0.1% by Bonferroni/Dunn test.

The percentage of the viable cell in the absence of the added amino acid before the UV-A 9 J/cm² irradiation (negative control) was 24%. The percentage of the viable cell in the presence of the added D-proline at 0.1µM (micro-molar) before the irradiation (positive control) was 100%, showing the suppression of the cell death. Pre-irradiation addition of D-methionine at 0.01 µM (micro-molar), 0.1 µM (micro-molar), 1 µM (micro-molar), 10 µM (micro-molar) or 100 µM (micro-molar) resulted in the percentage of the viable cell of 102%, 81%, 97%, 114% or 76%, respectively. Pre-irradiation addition of L-methionine at 0.001 µM (micro-molar), 0.01 µM (micro-molar), 0.1 µM (micro-molar), 1 µM (micro-molar), 10 µM (micro-molar) or 100 µM (micro-molar) resulted in the percentage of the viable cell of 40%, 72%, 67%, 45%, 73% or 62%. Based on these results, the addition of L- or D-methionine resulted in an increased percentage of the viable cell and a reduced cell death.

### Results of pre-irradiation addition of methionine (2)

Figure 2 shows the results of the experiment examining the effect of D-methionine on the damage in the fibroblast induced by the ultraviolet irradiation with the UV-A at 9 J/cm². The error bars for relevant experimental conditions are the standard deviations of the measured values of the results of the experiments repeated three to four times under the same conditions. The asterisk (*) indicates p<5% by Bonferroni/Dunn test.

The percentage of the viable cell without UV irradiation and without added amino acid (hereinafter, referred to as "UV non-irradiation") was 100%. The percentage of the viable cell in the absence of the added amino acid before the UV-A 9 J/cm² irradiation (negative control) was 69%. The percentage of the viable cell in the presence of the added D-proline at 0.1µM before the irradiation (positive control) was 88%, showing the reduction of the cell death. Pre-irradiation addition of D-methionine at 0.0001 µM (micro-molar) and 0.1 µM (micro-molar) resulted in the percentage of the viable cell of 50% and 101%. Based on these results, the addition of D-methionine at 0.0001 µM (micro-molar) resulted in no increase in percentage of the viable cells, but addition of D-methionine at 0.1 µM (micro-molar) resulted in an increased percentage of the viable cell and a reduced cell death.

### Results of post-irradiation addition of methionine

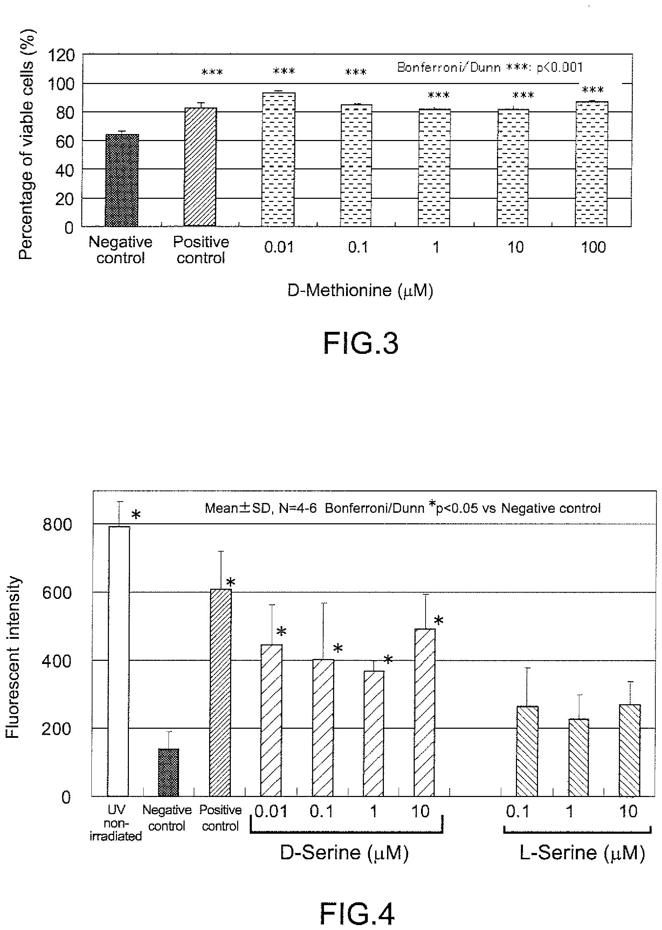

Figure 3 shows the results of the experiment examining the effect of D-methionine on the damage in the fibroblast induced by the ultraviolet irradiation with the UV-A at 8 J/cm². The error bars for relevant experimental conditions are the standard deviations of the measured values of the results of the experiments repeated four times under the same conditions. The asterisk (***) indicates p<0.1% by Bonferroni/Dunn test.

The percentage of the viable cell in the absence of the added amino acid after the UV-A 8 J/cm² irradiation (negative control) was 64%. The percentage of the viable cell in the presence of D-proline added at 0.1 µM (micro-molar) after the irradiation was 82%, showing the reduced cell death. Post-irradiation addition of D-methionine at 0.01 µM (micro-molar), 0.1 µM (micro-molar), 1 µM (micro-molar), 10 µM (micro-molar) or 100 µM (micro-molar) resulted in the percentage of the viable cell of 93%, 84%, 82%, 81% or about 87%. Based on these results, the addition of D-methionine resulted in an increased percentage of the viable cell and a reduced cell death. It was also revealed that the cell death reducing effect was observed regardless of the time whether before or after the UV irradiation D-methionine was added. In addition, L-methionine also reduced the cell death induced by the UV irradiation. Accordingly, it was suggested that there is no relevancy to the time whether before or after the UV irradiation the L-methionine was added.

### Example 2 (not according to the invention)

### Alleviating effect of serine on ultraviolet irradiation-induced damage

### Methods

The cell culture, the addition of amino acids before the UV irradiation, the UV irradiation, the addition of amino acids after the UV irradiation and the cell damage measurement were conducted similarly to Example 1. The ultraviolet light (UV-A) was irradiated at 7.5 or 9 J/cm². For determining the effect of adding serine before ultraviolet irradiation (hereinafter, referred to as "pre-irradiation addition of serine") and the effect of adding serine after ultraviolet irradiation (hereinafter, referred to as "post-irradiation addition of serine"), L-serine (197-00403, Wako Pure Chemical Industries Ltd.) and D-serine (197-08823, Wako Pure Chemical Industries Ltd.) were employed at 0.0001 to 100 µM (micro-molar). The effect of the post-irradiation addition of serine was evaluated by irradiating the cell with 7.5 J/cm² of UV-A followed by switching back to the normal medium in which the culture was conducted for 21 hours and adding D-serine to this 21-hour cultured medium.

### Results of pre-irradiation addition of serine (1)

Figure 4 shows the results of the experiment examining the effect of the pre-irradiation addition of L- serine and D-serine on the damage in the fibroblast induced by the ultraviolet irradiation with the UV-A at 9 J/cm². The error bars for relevant experimental conditions are the standard deviations of the measured values of the results of the experiments repeated four to six times under the same conditions. The asterisk (*) indicates p<5% by Bonferroni/Dunn test.

The fluorescent intensity of Alamar Blue (trade mark) without ultraviolet irradiation was 794. The fluorescent intensity without added amino acid before the UV-A irradiation at 9 J/cm² (negative control) was 140. The fluorescent intensity with added D-proline at 0.1 µM (micro-molar) before the irradiation (positive control) was as high as 610 which indicated a reduced cell damage. The fluorescent intensities with added D-serine at 0.01 µM (micro-molar), 0.1 µM (micro-molar), 1 µM (micro-molar) and 10 µM (micro-molar) before the irradiation were 445, 402, 371 and 491, respectively. The fluorescent intensities with added L-serine at 0.1 µM (micro-molar), 1 µM (micro-molar) and 10 µM (micro-molar) before the irradiation were 265, 227 and 270, respectively. Based on these results, the pre-irradiation addition of L-serine resulted in almost no reduction in the cell damage. Meanwhile, the pre-irradiation addition of D-serine resulted in a statistically significant increase in the fluorescent intensity, showing a reduction in the cell damage.

### Results of pre-irradiation addition of serine (2)

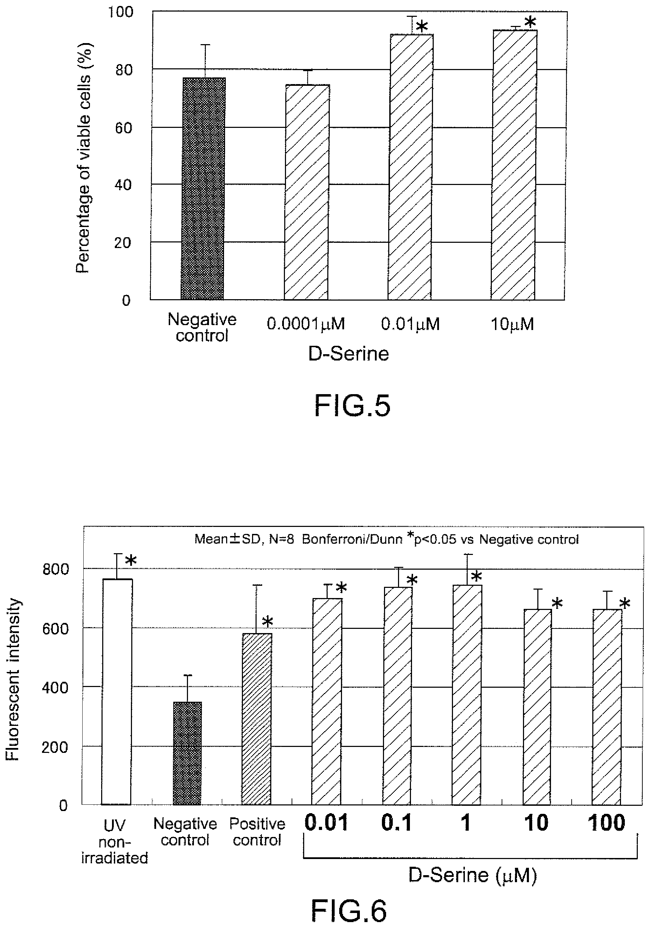

Figure 5 shows the results of the experiment examining the effect of the pre-irradiation addition of D-serine on the damage in the fibroblast induced by the ultraviolet irradiation with the UV-A at 8 J/cm². The error bars for relevant experimental conditions are the standard deviations of the measured values of the results of the experiments repeated four times under the same conditions. The asterisk (*) indicates p<5% by Bonferroni/Dunn test.

The percentage of the viable cell without ultraviolet irradiation was 100%. The percentage of the viable cell in the absence of the added amino acid before the UV-A 8 J/cm² irradiation (negative control) was 77%. The percentages of the viable cell in the presence of the added D-serine at 0.0001 µM (micro-molar), 0.01 µM (micro-molar) and 10 µM (micro-molar) before the irradiation were 74%, 92% and 93%, respectively. Based on these results, the ultraviolet-induced cell damage was characterized by the fact that the addition of D-serine at 0.0001 µM (micro-molar) resulted in an increased percentage of the viable cell and the addition of D-serine at 0.1 µM (micro-molar) and 10 µM (micro-molar) resulted in an increased percentage of the viable cell, indicating a reduced cell death.

### Results of post-irradiation addition of serine

Figure 6 shows the results of the experiment examining the effect of the post-irradiation addition of D-serine on the damage of the fibroblast induced by the ultraviolet irradiation with the UV-A at 7.5 J/cm². The error bars for relevant experimental conditions are the standard deviations of the measured values of the results of the experiments repeated eight times under the same conditions. The asterisk (*) indicates p<5% by Bonferroni/Dunn test.

The fluorescent intensity of Alamar Blue (trade mark) without ultraviolet irradiation was 764. The fluorescent intensity without added amino acid after the UV-A irradiation at 7.5 J/cm² (negative control) was 348. The fluorescent intensity with addition of D-proline at 0.1 µM (micro-molar) after the irradiation (positive control) was as high as 579 which indicated a reduced cell damage. The fluorescent intensities with addition of D-serine at 0.01 µM (micro-molar), 0.1 µM (micro-molar), 1 µM (micro-molar), 10 µM (micro-molar) and 100 µM (micro-molar) after the irradiation were 697, 735, 742, 664 and 663, respectively. Based on these results, the post-irradiation addition of D-serine resulted in a statistically significant increase in the fluorescent intensity, showing a reduction of the cell damage. It was also revealed that the cell damage reducing effect was obtained regardless of the time whether before or after the UV irradiation the D-serine was added.

### Example 3 (not according to the invention)

### Alleviating effect of D-cycloserine on ultraviolet irradiation-induced damage

### Methods

The cell culture, the addition of amino acids before the UV irradiation, the UV irradiation, the addition of amino acids after the UV irradiation and the cell damage measurement were conducted similarly to Example 1. The ultraviolet light (UV-A) was irradiated at 9 J/cm². For determining the effect of adding D-cycloserine before ultraviolet irradiation (hereinafter, referred to as "pre-irradiation addition of cycloserine"), D-cycloserine (C6880, Sigma) was employed at 0.0001 to 100 µM.

### Results of pre-irradiation addition of D-cycloserine

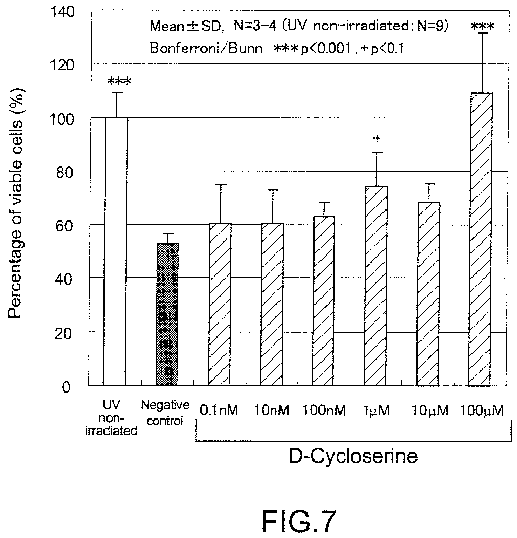

Figure 7 shows the results of the experiment examining the effect of the pre-irradiation addition of D-cycloserine on the damage of the fibroblast induced by the ultraviolet irradiation with the UV-A at 9 J/cm². The error bars for relevant experimental conditions are the standard deviations of the measured values of the results of the experiments repeated three to four times under the same conditions. The symbol (+) and the asterisk (***) indicate p<10% and p<0.1%, respectively, by Bonferroni/Dunn test.

The percentage of the viable cell without addition of amino acid before the UV-A irradiation at 9 J/cm² (negative control) was 53%. The percentage of the viable cell with added D-cycloserine at 0.1 nM, 10 nM, 100 nM, 1 µM (micro-molar), 10 µM (micro-molar) and 100 µM (micro-molar) before the irradiation were 60%, 60%, 63%, 74%, 69% and 109%. Based on these results, the addition of D-cycloserine resulted in an increased percentage of the viable cell, indicating a reduced cell death.

Based on the present invention, the formulation examples comprising D-methionine an emulsion formulation, a patch formulation, a tablet, a soft capsule, a granule, beverage, a candy, a cookie, miso paste, a French dressing, a mayonnaise, a French bread, a soy sauce, yogurt, dried seasoning powder for rice, seasoning sauce/sauce for natto(Japanese fermented soybean paste), natto, unrefined black vinegar, cream, body cream, a gel formulation, a peel-off mask, a wet pack, an emulsion, a face lotion and an aerosol formulation are shown below. Methionine in the following Formulation Examples was in D-form. These formulation examples are listed only for the purpose of exemplification and not intended to limit the scope of the invention.

### Formulation Example 1 (Emulsion formulation)

| (Composition) | Content (% by mass) |
|---|---|
| Methionine | 0.42 |
| Behenyl alcohol | 0.2 |
| Cetanol | 0.5 |
| Glycerin mono fatty acid ester | 1.8 |
| Hardened castor oil POE (60) | 1.0 |
| White petrolatum | 2.0 |
| Liquid paraffin | 10.0 |
| Isopropyl myristate | 3.0 |
| Methyl polysiloxane (6cs) | 1.5 |
| Conc. Glycerin | 13.0 |
| Dipropylene glycol | 2.0 |
| Carboxyvinyl polymer | 0.25 |
| Sodium hyaluronate | 0.005 |
| Potassium hydroxide | As appropriate |
| Lactic acid | As appropriate |
| Sodium edetate | As appropriate |
| Ethylparaben | As appropriate |

| Purified water | Remainder |
|---|---|
| | 100.00 |

### Formulation Example 2 (Patch formulation)

| (Composition) | Content (% by mass) |
|---|---|
| Methionine | 0.3 |
| Polyacrylic acid | 3.0 |
| Sodium polyacrylate | 2.5 |
| Gelatin | 0.5 |
| Sodium carboxymethyl cellulose | 4.0 |
| Polyvinyl alcohol | 0.3 |
| Conc. Glycerin | 14.0 |
| 1,3-Butylene glycol | 12.0 |
| Aluminum hydroxide | 0.1 |
| Sodium edetate | 0.03 |
| Methylparaben | 0.1 |
| Purified water | Remainder |
| | 100.00 |

### Formulation Example 3 (Tablet) (not according to the invention)

| (Composition) | Content (mg/tablet) |
|---|---|
| D-serine and/or D-cycloserine | 360.5 |
| Lactose | 102.4 |
| Calcium carboxymethyl cellulose | 29.9 |
| Hydroxypropyl cellulose | 6.8 |
| Magnesium stearate | 5.2 |
| Crystalline cellulose | 10.2 |
| | 515.0 |

### Formulation Example 4 (Tablet) (not according to the invention)

| (Composition) | Content (mg/tablet) |
|---|---|
| Sucrose ester | 70 |
| Crystalline cellulose | 74 |
| Methyl cellulose | 36 |
| Glycerin | 25 |
| D-serine and/or D-cycloserine | 475 |
| N-Acetylglucosamine | 200 |
| Hyaluronic acid | 150 |
| Vitamin E | 30 |
| Vitamin B6 | 20 |
| Vitamin B2 | 10 |
| α(alpha)-Lipoic acid | 20 |
| Coenzyme Q10 | 40 |
| Ceramide (Konjac extract) | 50 |
| L-proline | 300 |
| | 1500 |

### Formulation Example 5 (Soft capsule) (not according to the invention)

| (Composition) | Content (mg/capsule) |
|---|---|
| Edible soybean oil | 530 |
| Eucommia ulmoides extract | 50 |
| Ginseng extract | 50 |
| D-serine and/or D-cycloserine | 100 |
| Royal jelly | 50 |
| Maca | 30 |
| GABA | 30 |
| Beeswax | 60 |
| Gelatin | 375 |
| Glycerin | 120 |
| Glycerin fatty acid ester | 105 |
| | 1500 |

### Formulation Example 6 (Soft capsule) (not according to the invention)

| (Composition) | Content (mg/capsule) |
|---|---|
| Brown rice germ oil | 659 |
| D-serine and/or D-cycloserine | 500 |
| Resveratrol | 1 |
| Lotus germ extract | 100 |
| Elastin | 180 |
| DNA | 30 |
| Folic acid | 30 |
| | 1500 |

### Formulation Example 7 (Granule) (not according to the invention)

| (Composition) | Content (mg/pack) |
|---|---|
| D-serine and/or D-cycloserine | 400 |
| Vitamin C | 100 |
| Soybean isoflavon | 250 |
| Reduced lactose | 300 |
| Soybean oligosaccharide | 36 |
| Erythritol | 36 |
| Dextrin | 30 |
| Flavoring agent | 24 |
| Citric acid | 24 |
| | 1200 |

### Formulation Example 8 (Beverage) (not according to the invention)

| (Composition) | Content (g/60 mL) |
|---|---|
| Eucommia ulmoides extract | 1.6 |
| Ginseng extract | 1.6 |
| D-serine and/or D-cycloserine | 1.6 |
| Reduced maltose syrup | 28 |
| Erythritol | 8 |
| Citric acid | 2 |
| Flavoring agent | 1.3 |
| N-Acetylglucosamine | 1 |
| Sodium hyaluronate | 0.5 |
| Vitamin E | 0.3 |
| Vitamin B6 | 0.2 |
| Vitamin B2 | 0.1 |
| α(alpha)-Lipoic acid | 0.2 |
| Coenzyme Q10 | 1.2 |
| Ceramide (Konjac extract) | 0.4 |
| L-Proline | 2 |
| Purified water | Remainder |
| | 60 |

### Formulation Example 9 (Candy) (not according to the invention)

| (Composition) | Content (% by mass) |
|---|---|
| Sugar | 50 |
| Syrup | 48 |
| D-serine and/or D-cycloserine | 1 |
| Flavoring agent | 1 |
| | 100 |

### Formulation Example 10 (Cookie) (not according to the invention)

| (Composition) | Content (% by mass) |
|---|---|
| Weak flour | 45.0 |
| Butter | 17.5 |
| Granulated sugar | 20.0 |
| D-serine and/or D-cycloserine | 4.0 |
| Egg | 12.5 |
| Flavoring agent | 1.0 |
| | 100.0 |

### Method for producing Formulation Example 10 (Cookie)

Granular sugar was added in portions to butter while stirring, to which an egg, D-serine and/or D-cycloserine together with a flavoring agent were added and stirred. After mixing thoroughly, uniformly sieved weak flour was added and then stirred slowly, and allowed to stand as a bulk in a refrigerator. Thereafter, it was molded and baked for 15 minutes at 170°C (degrees Celsius) to obtain a cookie.

### Formulation Example 11 (Miso (soybean) paste) (not according to the invention)

| (Composition) | Content (g) |
|---|---|
| Soybean | 1000 |
| Malted rice | 1000 |
| Salt | 420 |
| D-serine and/or D-cycloserine | 158 |
| Water | Remainder |
| | 4000 |

### Method for producing Formulation Example 11 (Miso (soybean) paste)

Malted rice is mixed thoroughly with a salt. Washed soybeans are soaked in three times its volume of water, which are then drained off, and new water is added while boiling, and poured into a colander to collect the broth (tanemizu fluid), to which D-serine and/or D-cycloserine is dissolved at 10% w/v. The boiled beans are minced immediately, combined with malted rice mixed with salt, to which the tanemizu fluid described above containing D-serine and/or D-cycloserine dissolved therein is added and kneaded evenly to obtain a clay-like hardness. Dumplings are made and stuffed in a container compactly without forming any void, and the surface of the content is smoothened and sealed by wrapping with a plastic film. After three months, the content is transferred to a new container and the surface is smoothened and sealed by wrapping with a plastic film. Instead of adding D-serine and/or D-cycloserine to the tanemizu fluid, a malted rice producing a large amount of D-serine and/or D-cycloserine may be employed. Such malted rice can be selected by quantifying D-serine and/or D-cycloserine by the method described in Japanese Patent Unexamined Publication No. 2008-185558. Alternatively, a commercially available miso paste can be supplemented with D-serine and/or D-cycloserine or a salt thereof.

### Formulation Example 12 (French dressing) (not according to the invention)

| (Composition) | Content (g) |
|---|---|
| Salad oil | 27.0 |
| Vinegar | 30.0 |
| Sodium chloride | 0.9 |
| D-serine and/or D-cycloserine | 1.1 |
| Pepper | 1.0 |
| | 60.0 |

### Method for producing Formulation Example 12 (French dressing)

Vinegar is combined with sodium chloride as well as D-serine and/or D-cycloserine, stirred thoroughly and then a pepper is added.

### Formulation Example 13 (Mayonnaise) (not according to the invention)

| (Composition) | Content (g) |
|---|---|
| Salad oil | 134.0 |
| Vinegar | 5 |
| Sodium chloride | 0.9 |
| D-serine and/or D-cycloserine | 1 |
| Egg yolk | 18 |
| Sugar | 0.2 |
| Pepper | 0.9 |
| | 160.0 |

### Method for producing Formulation Example 13 (Mayonnaise)

An egg yolk (room temperature) is combined with vinegar, sodium chloride and pepper as well as D-serine and/or D-cycloserine, and stirred thoroughly using a whipping apparatus. Stirring is continued while adding salad oil in portions to form an emulsion. Finally, a sugar is added and the mixture is stirred.

### Formulation Example 14 (French bread) (not according to the invention)

| (Composition) | Content (g) |
|---|---|
| Hard flour | 140 |
| Weak flour | 60 |
| Sodium chloride | 3 |
| Sugar | 6 |
| D-serine and/or D-cycloserine | 2 |
| Dry yeast | 4 |
| Lukewarm water | 128 |
| | 343 |

### Method for producing Formulation Example 14 (French bread)

Lukewarm water is combined with 1 g of sugar and dry yeast, which is then allowed to undergo a pre-fermentation. Hard flour, weak flour, sodium chloride and 5 g of sugar are placed in a bowl together with D-serine and/or D-cycloserine, into which the pre-fermented yeast is placed. After kneading thoroughly into a ball-like dough, a primary fermentation is conducted at 30°C. The dough is kneaded again and allowed to stand, and then shaped into suitable forms, which are subjected to a final fermentation using an electronic fermentation machine. After forming coupes, baking is conducted for 30 minutes in an oven at 220°C (degrees Celsius).

### Formulation Example 15 (Soy sauce) (not according to the invention)

| (Composition) | Content (g) |
|---|---|
| Commercially available soy sauce | 900 |
| D-serine and/or D-cycloserine | 100 |
| | 1000 |

### Method for producing Formulation Example 15 (Soy sauce)

Commercially available soy sauce is supplemented with D-serine and/or D-cycloserine, and stirred thoroughly. Instead of adding D-serine and/or D-cycloserine or a salt thereof, malted rice producing a large amount of D-serine and/or D-cycloserine may be employed for fermenting soy sauce. Such malted rice can be selected by quantifying D-serine and/or D-cycloserine by the method described in Japanese Patent Unexamined Publication No. 2008-185558.

### Formulation Example 16 (Yogurt) (not according to the invention)

| (Composition) | Content (g) |
|---|---|
| Milk | 880 |
| L.bulgaricus | 50 |
| S.thermophilus | 50 |
| D-serine and/or D-cycloserine | 20 |
| | 1000 |

### Method for producing Formulation Example 16 (Yogurt)

Fermentation is conducted at 40°C (degrees Celsius) to 45°C (degrees Celsius). Other commercially available fermentation seed organisms may be employed and commercially available yogurt may be supplemented with D-serine and/or D-cycloserine. Instead of adding D-serine and/or D-cycloserine or a salt thereof, an organism producing a large amount of D-serine and/or D-cycloserine may be employed for fermentation. Such an organism can be selected by quantifying D-serine and/or D-cycloserine by the method described in Japanese Patent Unexamined Publication No. 2008-185558.

### Formulation Example 17 (Dried seasoning powder for rice) (not according to the invention)

| (Composition) | Content (g) |
|---|---|
| D-serine and/or D-cycloserine | 50 |
| Laver | 15 |
| Sodium L-glutamate | 10 |
| Sodium chloride | 2 |
| Roasted sesame | 10 |
| Dried mackerel flakes | 10 |
| Sugar | 1 |
| Soy sauce | 2 |
| | 100 |

### Formulation Example 18 (Seasoning, Sauce for natto) (not according to the invention)

| (Composition) | Content (g) |
|---|---|
| Commercially available sauce | 9 |
| for natto | |
| D-serine and/or D-cycloserine | 1 |
| | 10 |

### Formulation Example 19 (Natto) (not according to the invention)

| (Composition) | Content (g) |
|---|---|
| Commercially available natto | 19.9 |
| D-serine and/or D-cycloserine | 0.1 |
| | 20 |

### Method for producing Formulation Example 19 (Natto)

Instead of adding D-serine and/or D-cycloserine or a salt thereof, an organism producing a large amount of D-serine and/or D-cycloserine may be employed for producing natto. Such an organism can be selected by quantifying D-serine and/or D-cycloserine by the method described in Japanese Patent Unexamined Publication No. 2008-185558.

### Formulation Example 20 (Unrefined black vinegar) (not according to the invention)

| | |
|---|---|
| (Composition) | Content (g) |
| Commercially available unrefined black vinegar | 900 |
| D-serine and/or D-cycloserine | 100 |
| | 1000 |

### Method for producing Formulation Example 20 (Unrefined black vinegar)

Instead of adding D-serine and/or D-cycloserine or a salt thereof, an organism producing a large amount of D-serine and/or D-cycloserine may be employed for producing vinegar, black vinegar or unrefined vinegar. Such an organism can be selected by quantifying D-serine and/or D-cycloserine by the method described in Japanese Patent Unexamined Publication No. 2008-185558.

### Formulation Example 21 (Cream)

| (Composition) | Content (%) |
|---|---|
| Liquid paraffin | 3 |
| Petrolatum | 1 |
| Dimethyl polysiloxane | 1 |
| Stearyl alcohol | 1.8 |
| Behenyl alcohol | 1.6 |
| Glycerin | 8 |
| Dipropylene glycol | 5 |
| Macadamia nut oil | 2 |
| Hardened oil | 3 |
| Squalane | 6 |
| Stearic acid | 2 |
| Cholesteryl hydroxystearate | 0.5 |
| Cetyl 2-ethylhexanoate | 4 |
| Polyoxyethylene hardened castor oil | 0.5 |
| Self-emulsified glycerin monostearate | 3 |
| Potassium hydroxide | 0.15 |
| Sodium hexametaphosphate | 0.05 |
| Trimethyl glycine | 2 |
| L-ascorbic acid dl-alpha -tocopherol phosphoric acid diester potassium salt | 1 |
| Tocopherol acetate | 0.1 |
| Methionine | 4 |
| Paraben | As appropriate |
| Trisodium edetate | 0.05 |
| 4-t-Butyl-4'-methoxybenzoyl methane | 0.05 |
| Glyceryl diparamethoxycinnamate mono-2-ethylhexanoate | 0.05 |
| Colorant | As appropriate |
| Carboxyvinyl polymer | 0.05 |
| Purified water | Remainder |
| | 100.00 |

### Formulation Example 22 (Body cream) (not according to the invention)

| (Composition) | Content (% by mass) |
|---|---|
| Dimethyl polysiloxane | 3 |
| Decamethyl cyclopentasiloxane | 13 |
| Dodecamethyl cyclohexasiloxane | 12 |
| Polyoxyethylene - methyl polysiloxane copolymer | 1 |
| Ethanol | 2 |
| Isopropanol | 1 |
| Glycerin | 3 |
| Dipropylene glycol | 5 |
| Polyethylene glycol 6000 | 5 |
| Sodium hexametaphosphate | 0.05 |
| Tocopherol acetate | 0.1 |
| D-serine and/or D-cycloserine | 5 |
| Fennel extract | 0.1 |
| Witch hazel extract | 0.1 |
| Ginseng extract | 0.1 |
| L-Menthol | As appropriate |
| p-oxybenzoate | As appropriate |
| Trisodium edetate | 0.05 |
| Dimorpholinopyridazinone | 0.01 |
| Methylbis(trimethylsiolxy)s ilylisopentyl trimethoxycinnamate | 0.1 |
| Iron oxide yellow | As appropriate |
| Cobalt titanate | As appropriate |
| Dimethyl distearyl ammonium hectolite | 1.5 |
| Polyvinyl alcohol | 0.1 |
| Hydroxyethyl cellulose | 0.1 |
| Trimethylsiloxy silicic acid | 2 |
| Fragrance | As appropriate |
| Purified water | Remainder |
| | 100.00 |

### Formulation Example 23 (Body cream)

| (Composition) | Content (% by mass) |
|---|---|
| Dimethyl polysiloxane | 3 |
| Decamethyl cyclopentasiloxane | 13 |
| Dodecamethyl cyclohexasiloxane | 12 |
| Polyoxyethylene - methyl polysiloxane copolymer | 1 |
| Ethanol | 2 |
| Isopropanol | 1 |
| Glycerin | 3 |
| Dipropylene glycol | 5 |
| Polyethylene glycol 6000 | 5 |
| Sodium hexametaphosphate | 0.05 |
| Tocopherol acetate | 0.1 |
| Methionine | 3 |
| Fennel extract | 0.1 |
| Witch hazel extract | 0.1 |
| Ginseng extract | 0.1 |
| L-Menthol | As appropriate |
| p-oxybenzoate | As appropriate |
| Trisodium edetate | 0.05 |
| Dimorpholinopyridazinone | 0.01 |
| Isopentyl trimethoxycinnamate trisiloxane | 0.1 |
| Iron oxide yellow | As appropriate |
| Cobalt titanate | As appropriate |
| Dimethyl distearyl ammonium hectolite | 1.5 |
| Polyvinyl alcohol | 0.1 |
| Hydroxyethyl cellulose | 0.1 |
| Trimethylsiloxysilicate | 2 |
| Fragrance | As appropriate |
| Purified water | Remainder |
| | 100.00 |

### Formulation Example 24 (Gel formulation)

| (Composition) | Content (% by mass) |
|---|---|
| Dimethyl polysiloxane | 5 |
| Glycerin | 2 |
| 1,3-Butylene glycol | 5 |
| Polyethylene glycol 1500 | 3 |
| Polyethylene glycol 20000 | 3 |
| Cetyl octanoate | 3 |
| Citric acid | 0.01 |
| Sodium citrate | 0.1 |
| Sodium hexametaphosphate | 0.1 |
| Dipotassium glycyrrhizinate | 0.1 |
| Methionine | 2 |
| Tocopherol acetate | 0.1 |
| Scutellaria root extract | 0.1 |
| Saxifrage extract | 0.1 |
| Trisodium edetate | 0.1 |
| Xanthane gum | 0.3 |
| Acrylates/C10-30 alkyl acrylate crosspolymer (Pemulen TR-2) | 0.05 |
| Agar powder | 1.5 |
| Phenoxyethanol | As appropriate |
| Dibutylhydroxytoluene | As appropriate |
| Purified water | Remainder |
| | 100.00 |

### Formulation Example 25 (Peel-off mask) (not according to the invention)

| (Composition) | Content (% by mass) |
|---|---|
| Ethanol | 10 |
| 1,3-Butylene glycol | 6 |
| Polyethylene glycol 4000 | 2 |
| Olive oil | 1 |
| Macadamia nut oil | 1 |
| Phytosteryl hydroxystearic acid | 0.05 |
| Lactic acid | 0.05 |
| Sodium lactate | 0.1 |
| Disodium L-ascorbate sulfate | 0.1 |
| L-ascorbic acid dl-alpha -tocopherol phosphoric acid diester potassium salt | 0.1 |
| D-serine and/or D-cycloserine | 10 |
| Fish collagen | 0.1 |
| Sodium chondroitin sulfate | 0.1 |
| Sodium carboxymethyl cellulose | 0.2 |
| Polyvinyl alcohol | 12 |
| p-oxybenzoate | As appropriate |
| Fragrance | As appropriate |
| Purified water | Remainder |
| | 100.00 |

### Formulation Example 26 (Peel-off mask)

| (Composition) | Content (% by mass) |
|---|---|
| Ethanol | 10 |
| 1,3-Butylene glycol | 6 |
| Polyethylene glycol 4000 | 2 |
| Olive oil | 1 |
| Macadamia nut oil | 1 |
| Phytosteryl hydroxystearic acid | 0.05 |
| Lactic acid | 0.05 |
| Sodium lactate | 0.1 |
| Disodium L-ascorbate sulfate | 0.1 |
| L-ascorbic acid dl-alpha -tocopherol phosphoric acid diester potassium salt | 0.1 |
| Methionine | 4 |
| Fish collagen | 0.1 |
| Sodium chondroitin sulfate | 0.1 |
| Sodium carboxymethyl cellulose | 0.2 |
| Polyvinyl alcohol | 12 |
| p-oxybenzoate | As appropriate |
| Fragrance | As appropriate |
| Purified water | Remainder |
| | 100.00 |

### Formulation Example 27 (Wet pack)

| (Composition) | Content (% by mass) |
|---|---|
| Glycerin | 1 |
| 1,3-Butylene glycol | 8 |
| Xylit | 2 |
| Polyethylene glycol 1500 | 2 |
| Rosemary oil | 0.01 |
| Sage oil | 0.1 |
| Citric acid | 0.02 |
| Sodium citrate | 0.08 |
| Sodium hexametaphosphate | 0.01 |
| Hydroxypropyl-β (beta)-cyclodextrin | 0.1 |
| Methionine | 0.5 |
| Birch extract | 0.1 |
| Lavender extract | 0.01 |
| Xanthane gum | 0.05 |
| Carboxyvinyl polymer | 0.15 |
| p-oxybenzoate | As appropriate |
| Purified water | Remainder |
| | 100.00 |

### Formulation Example 28 (Emulsion)

| (Composition) | Content (% by mass) |
|---|---|
| Liquid paraffin | 7 |
| Petrolatum | 3 |
| Decamethyl cyclopentasiloxane | 2 |
| Behenyl alcohol | 1.5 |
| Glycerin | 5 |
| Dipropylene glycol | 7 |
| Polyethylene glycol 1500 | 2 |
| Jojoba oil | 1 |
| Isostearic acid | 0.5 |
| Stearic acid | 0.5 |
| Behenic acid | 0.5 |
| Pentaerythritol tetra (2-ethylhexanoate) | 3 |
| Cetyl 2-ethylhexanoate | 3 |
| Glycerin monostearate | 1 |
| Polyoxyethylene-glycerin | 1 |
| monostearate | |
| Potassium hydroxide | 0.1 |
| Sodium hexametaphosphate | 0.05 |
| Stearyl glycyrrhizinate | 0.05 |
| Methionine | 1 |
| Royal jelly extract | 0.1 |
| Yeast extract | 0.1 |
| Tocopherol acetate | 0.1 |
| Acetylated sodium hyaluronate | 0.1 |
| Trisodium edetate | 0.05 |
| 4-t-Butyl-4'-methoxydibenzoy lmethane | 0.1 |
| 2-Ethylhexyl paramethoxycinnamate | 0.1 |
| Carboxyvinyl polymer | 0.15 |
| Paraben | As appropriate |
| Fragrance | As appropriate |
| Purified water | Remainder |
| | 100.00 |

Formulation Example 29 (Emulsion)

| (Composition) | Content (% by mass) |
|---|---|
| Dimethylpolysiloxane | 2 |
| Behenyl alcohol | 1 |
| Batyl alcohol | 0.5 |
| Glycerin | 5 |
| 1,3-Butylene glycol | 7 |
| Erythritol | 2 |
| Hardened oil | 3 |
| Squalane | 6 |
| Pentaerythritol tetra (2-ethylhexanoate) | 2 |
| Polyoxyethylene glyceryl isostearate | 1 |
| Polyoxyethylene glyceryl monostearate | 1 |
| Methionine | 0.3 |
| Potassium hydroxide | As appropriate |
| Sodium hexametaphosphate | 0.05 |
| Phenoxyethanol | As appropriate |
| Carboxyvinyl polymer | 0.1 |
| Purified water | Remainder |
| | 100.00 |

### Formulation Example 30 (Skin lotion) (not according to the invention)

| (Composition) | Content (% by mass) |
|---|---|
| Ethyl alcohol | 5 |
| Glycerin | 1 |
| 1,3-Butylene glycol | 5 |
| Polyoxyethylene-polyoxypropylene decyltetradecyl ether | 0.2 |
| Sodium hexametaphosphate | 0.03 |
| Trimethyl glycine | 1 |
| Sodium polyaspartic acid | 0.1 |
| L-ascorbic acid dl-alpha -tocopherol phosphoric acid diester potassium salt | 0.1 |
| Thiotaurine | 0.1 |
| D-serine and/or D-cycloserine | 8 |
| Trisodium EDTA | 0.1 |
| Carboxyvinyl polymer | 0.05 |
| Potassium hydroxide | 0.02 |
| Phenoxyethanol | As appropriate |
| Fragrance | As appropriate |
| Purified water | Remainder |
| | 100.00 |

### Formulation Example 31 (Skin lotion)

| (Composition) | Content (% by mass) |
|---|---|
| Ethyl alcohol | 5 |
| Glycerin | 1 |
| 1,3-Butylene glycol | 5 |
| Polyoxyethylene-polyoxypropylene decyltetradecyl ether | 0.2 |
| Sodium hexametaphosphate | 0.03 |
| Trimethyl glycin | 1 |
| Sodium polyaspartic acid | 0.1 |
| L-ascorbic acid dl-alpha -tocopherol phosphoric acid diester potassium salt | 0.1 |
| Thiotaurine | 0.1 |
| Methionine | 4 |
| Trisodium EDTA | 0.1 |
| Carboxyvinyl polymer | 0.05 |
| Potassium hydroxide | 0.02 |
| Phenoxyethanol | As appropriate |
| Fragrance | As appropriate |
| Purified water | Remainder |
| | 100.00 |

### Formulation Example 32 (Skin lotion)

| (Composition) | Content (% by mass) |
|---|---|
| Ethanol | 10 |
| Dipropylene glycol | 1 |
| Polyethylene glycol 1000 | 1 |
| Polyoxyethylene methyl glucoside | 1 |
| Jojoba oil | 0.01 |
| Glyceryl tri(2-ethylhexanoate) | 0.1 |
| Polyoxyethylene hardened castor oil | 0.2 |
| Polyglyceryl diisostearate | 0.15 |
| Sodium N-stearoyl L-glutamate | 0.1 |
| Citric acid | 0.05 |
| Sodium citrate | 0.2 |
| Potassium hydroxide | 0.4 |
| Dipotassium glycyrrhizinate | 0.1 |
| Arginine hydrochloride | 0.1 |
| L-Ascorbic acid 2-glucoside | 2 |
| Methionine | 0.5 |
| Trisodium edetate | 0.05 |
| 2-Ethylhexyl paramethoxycinnamate | 0.01 |
| Dibutylhydroxytoluene | As appropriate |
| Paraben | As appropriate |
| Deep seawater | 3 |
| Fragrance | As appropriate |
| Purified water | Remainder |
| | 100.00 |

### Formulation Example 33 (Stock solution for external preparation of aerosol urea)

| (Composition) | Content (% by mass) |
|---|---|
| Ethanol | 15.0 |
| Polyoxyethylene hardened castor oil 50 | 1.5 |
| Diphenhydramine | 1.0 |
| Dibucaine | 2.0 |
| Tocopherol acetate | 0.5 |
| Methionine | 0.1 |
| Isostearic acid | 0.1 |
| 1,3-Butylene glycol | 3.0 |
| Polyethylene glycol 400 | 3.0 |
| Camphor | 0.05 |
| Urea | 20.0 |
| Purified water | Remainder |
| | 100.00 |

### Formulation Example 34 (Aerosol urea spray)

| (Composition) | Content (% by mass) |
|---|---|
| (Stock solution for external preparation of aerosol urea) | 65.0 |
| Dimethyl ether | 35.0 |
| | 100.00 |

### Method for filling Formulation Example 34 (Aerosol urea spray)

Stock solution for external preparation of aerosol urea and dimethyl ether are charged in a pressure-resistant aluminum aerosol container whose inner surface is coated with Teflon (trade mark) to yield an aerosol preparation.

## Claims

1. A composition for use in preventing or treating an ultraviolet irradiation-induced skin disease, said composition comprising one or more compounds selected from a group consisting of D-methionine and/or salts thereof.

2. The composition for use according to claim 1, wherein the skin disease is selected from a group consisting of erythema, solar dermatitis, chronic actinic dermatopathy, actinic keratosis, actinic cheilitis, Favre-Racouchot disease, photodermatosis, photocontact dermatitis, berloque dermatitis, photosensitive drug eruption, polymorphous light eruption, hydroa vacciniforme, solar urticaria, chronic photosensitive dermatitis, xeroderma pigmentosum, ephelides, porphyria, pellagra, Hartnup disease, solar keratosis, dermatomyositis, lichen planus, Darier disease, pityriasis rubra pilaris, rosacea, atopic dermatitis, chloasma, herpes simplex, lupus erythematosus, squamous cell carcinoma, basal cell carcinoma and Bowen disease.

3. The composition for use according to claim 1 or 2, which is provided in the form of an external preparation for the skin or in the form of a food product.

4. Non-therapeutic use of a composition comprising one or more compounds selected from a group consisting of D-methionine and/or salts thereof as an agent for improving an ultraviolet irradiation-induced cosmetic skin condition.

5. The use according to claim 4, wherein the cosmetic skin condition is wrinkles.

6. Composition for use according to claim 1, wherein the composition is a sunscreen agent.

7. The use according to claim 4 or 5, or the composition for use according to claim 6, wherein the composition is provided in the form of an external preparation for the skin or in the form of a food product.

## Patentansprüche

1. Zusammensetzung zur Verwendung für die Prävention oder Behandlung einer durch ultraviolette Strahlung induzierten Hauterkrankung, wobei die Zusammensetzung eine oder mehrere Verbindungen ausgewählt aus der Gruppe bestehend aus D-Methionin und/oder Salzen hiervon umfasst.

2. Zusammensetzung zur Verwendung nach Anspruch 1, wobei die Hauterkrankung aus der Gruppe bestehend aus Erythem, Sonnendermatitis, chronischer aktinischer Dermatitis, aktinischer Keratose, aktinischer Cheilitis, Favre-Racouchot-Krankheit, Photodermatose, Photokontaktdermatitis, Berloque-Dermatitis, photosensitiver Arzneimitteldermatitis, polymorpher Lichtdermatose, Hydroa vacciniforme, Sonnenurtikaria, chronischer photosensitiver Dermatitis, Xeroderma pigmentosum, Sommersprossen, Porphyrie, Pellagra, Hartnup-Krankheit, Sonnenkeratose, Dermatomyositis, Lichen planus, Morbus Darier, Pityriasis rubra pilaris, Rosazea, atopischer Dermatitis, Chloasma, Herpes simplex, Lupus erythematodes, Plattenepithelkarzinom, Basalzellenkarzinom und Morbus Bowen ausgewählt ist.

3. Zusammensetzung zur Verwendung nach Anspruch 1 oder 2, welche in Form einer externen Zubereitung für die Haut oder in Form eines Nahrungsmittelprodukts bereitgestellt ist.

4. Nicht-therapeutische Verwendung einer Zusammensetzung, welche eine oder mehrere Verbindungen ausgewählt aus der Gruppe bestehend aus D-Methionin und/oder Salzen hiervon umfasst, als Mittel zur Verbesserung eines durch ultraviolette Strahlung induzierten kosmetischen Hautzustands.

5. Verwendung nach Anspruch 4, wobei es sich bei dem kosmetischen Hautzustand um Falten handelt.

6. Zusammensetzung zur Verwendung nach Anspruch 1, wobei es sich bei der Zusammensetzung um ein Sonnenschutzmittel handelt.

7. Verwendung nach Anspruch 4 oder 5, oder Zusammensetzung zur Verwendung nach Anspruch 6, wobei die Zusammensetzung in Form einer externen Zubereitung für die Haut oder in Form eines Nahrungsmittelprodukts bereitgestellt ist.

## Revendications

1. Composition destinée à être utilisée pour prévenir ou traiter une maladie de peau induite par un rayonnement ultraviolet, ladite composition comprenant un ou plusieurs composés choisis dans le groupe constitué par la D-méthionine et/ou ses sels.

2. Composition destinée à être utilisée selon la revendication 1, la maladie de la peau étant choisie dans le groupe constitué par l'érythème, la dermatite solaire, la dermatite actinique chronique, la kératose actinique, la chéilite actinique, la maladie de Favre et Racouchot, la photodermatose, la dermatite de photocontact, la dermatite en breloque, l'éruption médicamenteuse photosensible, l'éruption légère polymorphe, l'hydroa vacciniforme, l'urticaire solaire, la dermatite chronique photosensible, la xérodermie pigmentaire, les éphélides, la porphyrie cutanée, la pellagre, la maladie de Hartnup, la kératose solaire, la dermatomyosite, le lichen plan, la maladie de Darier, le pityriasis rubra pilaire, la rosacée, la dermatite atopique, le chloasma, l'herpès simplex, le lupus érythémateux, le carcinome à cellules squameuses, le carcinome à cellules basales et la maladie de Bowen.

3. Composition destinée à être utilisée selon la revendication 1 ou 2, qui est fournie sous la forme d'une préparation à usage externe pour la peau ou sous la forme d'un produit alimentaire.

4. Utilisation non thérapeutique d'une composition comprenant un ou plusieurs composés choisis dans le groupe constitué par la D-méthionine et/ou ses sels comme d'agent d'amélioration d'une affection cutanée cosmétique induite par un rayonnement ultraviolet.

5. Utilisation selon la revendication 4, l'affection cutanée cosmétique étant les rides.

6. Composition destinée à être utilisée selon la revendication 1, la composition étant un agent de type écran solaire.

7. Utilisation selon la revendication 4 ou 5, ou composition destinée à être utilisée selon la revendication 6, la composition étant fournie sous la forme d'une préparation à usage externe pour la peau ou sous la forme d'un produit alimentaire.
